(19) Europäisches Patentamt
European Patent Office
Office européen des brevets

(11) **EP 4 545 938 A1**

(12) **EUROPEAN PATENT APPLICATION**

(43) Date of publication:
**30.04.2025 Bulletin 2025/18**

(21) Application number: **23205809.9**

(22) Date of filing: **25.10.2023**

(51) International Patent Classification (IPC):
**G01N 17/00** (2006.01)   **G01N 17/04** (2006.01)
**G01N 33/00** (2006.01)

(52) Cooperative Patent Classification (CPC):
**G01N 17/04; G01N 17/006; G01N 33/0044**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **ABB SCHWEIZ AG**
**5400 Baden (CH)**

(72) Inventors:
- **Leppäniemi, Jaakko**
  **04130 Sipoo (FI)**
- **Mäkelä, Tapio**
  **00740 Helsinki (FI)**
- **Smolander, Maria**
  **02710 Espoo (FI)**

(74) Representative: **Kolster Oy Ab**
**Salmisaarenaukio 1**
**P.O. Box 204**
**00181 Helsinki (FI)**

(54) **CORROSION MONITORING**

(57)    A corrosion sensor is disclosed, comprising a corroding metal thin film encapsulated with an electrically insulating polymer layer, wherein the polymer layer is permeable to a corrosive gas by diffusion. An increase in the electrical resistance of the corrosion sensor, under exposure to corrosive gas that corrodes the corroding metal thin film when the corrosive gas permeates the polymer by diffusion, provides an indication of corrosion.

Figure 1

EP 4 545 938 A1

**Description**

FIELD OF THE INVENTION

The present invention relates to corrosion monitoring, and more particularly to a corrosion sensor.

BACKGROUND ART

The following description of background art may include insights, discoveries, understandings or disclosures, or associations together with disclosures not known to the relevant art prior to the present invention but provided by the invention. Some such contributions of the invention may be specifically pointed out below, whereas other such contributions of the invention will be apparent from their context.

Corrosion refers to deterioration of metal due to chemical reactions between the metal and the environment. Corrosion of electronic components occurs under ambient conditions containing corrosive gases, which may cause electronic component failure or electronic system failure, operation downtime, and expensive repairs.

The presence of corrosive gases at ppb-level concentrations can be detected using indirect measurements performed on corroding metal sheets, such as corrosion coupons(e.g. by Corrosion Classification Coupon by Purafil), wherein off-line analysis tools are used for detecting the layer thickness of corroded crust formed on top of a corroding metal sheet, such as Ag, Cu or Fe. The thickness of the corroded layer may then be correlated with a certain amount of corrosive gases.

In a visual inspection of Ag corrosion sensor, the silvery color of the Ag thin-film changes with respect to corrosive, sulfur-containing gases. The Ag corrosion sensor may be used to qualitatively distinguish between $H_2S$ and reduced sulfur gas ($S_8$) by the length of a blackened region of the corrosion sensor, due to the different diffusivity of the gases in air, as $H_2S$ leads to a longer black region due to its higher diffusivity. The concentration of the gas is qualitatively detected by the length of the yellow region of silver sulfide ($Ag_2S$).

However, such slow and cumbersome off-line measurements are not sufficient when needing to respond to rapid changes in the ambient gas. In addition, the off-line measurements are not useful for detecting a chemical composition or relative concentration of the corrosive gases.

For on-line continuous monitoring of corrosive gases, the Ag corrosion sensor may be used in an electrical corrosion sensor, where a thin film of Ag is deposited on top of non-corroding base metal. The corroded Ag metal changes the resistor network resistance of the base metal and Ag thin film connected in parallel.

SUMMARY

The following presents a simplified summary of features disclosed herein to provide a basic understanding of some exemplary aspects of the invention. This summary is not an extensive overview of the invention. It is not intended to identify key/critical elements of the invention or to delineate the scope of the invention. Its sole purpose is to present some concepts disclosed herein in a simplified form as a prelude to a more detailed description.

According to an aspect, there is provided the subject matter of the independent claims. Embodiments are defined in the dependent claims.

One or more examples of implementations are set forth in more detail in the description below. Other features will be apparent from the description, and from the claims.

BRIEF DESCRIPTION OF THE DRAWINGS

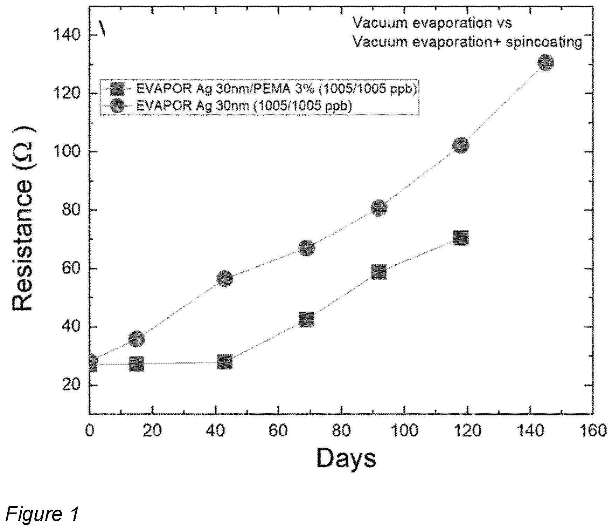

In the following the invention will be described in greater detail by means of preferred embodiments with reference to the attached drawings, in which
Figure 1 shows tests results regarding sensitivity obtained for polymer encapsulated metal thin film based corrosion sensors.

DETAILED DESCRIPTION OF EMBODIMENTS

The following embodiments are exemplary. Although the specification may refer to "an", "one", or "some" embodiment(s) in several locations, this does not necessarily mean that each such reference is to the same embodiment(s), or that the feature only applies to a single embodiment. Single features of different embodiments may also be combined to provide other embodiments. Furthermore, words "comprising", "containing" and "including" should be understood as not limiting the described embodiments to consist of only those features that have been mentioned and such embodiments may contain also features/structures that have not been specifically mentioned.

Although being a noble metal, silver may corrode in ambient atmosphere e.g. by reacting with sulfur-containing gases such as hydrogen sulfide ($H_2S$) and/or carbonyl sulfide. Copper is considered a noble metal; it resists attack by oxygen, although some air pollutants, such as $H_2S$, change its surface properties, even at ambient temperature. Copper sulphidation is a fast process occurring on the metal-gas phase interface impairing the corrosion resistance of copper, and leading to electric failures of electronic devices.

Permeation of gases through nonporous polymer film membranes follows a solution and diffusion mechanism when there is a pressure difference over the membrane. The first step in the gas permeation is a solution step where gas absorption occurs into the membrane from the interface at the higher-pressure side of the membrane. Then the gas diffuses through the membrane by molecular diffusion (via random walk), and finally the gas is desorbed at the interface on the lower-pressure side of the membrane. The molecular diffusion is a rate limiting step in the permeation. The rate of molecular diffusion may be estimated using Fick's laws of diffusion. When the gas diffuses through the membrane, there is a delay between the initial exposure of the gas on one side of the membrane and emergence of the gas on the other side of the membrane at a constant rate. This delay is given by a time lag (L), where $L = h^2/(6D_0)$, where h is the coating thickness in cm, and $D_0$ is the diffusion coefficient in $cm^2/s$.

When assuming Ag (or Cu) being an infinite sink for the diffusing corrosive gas (e.g. $H_2S$ reacting with Ag to yield $Ag_2S$), the delay between the onset of the corrosion of the metal film under the gas permeable coating may be assumed to follow a similar time lag. Thus, the sensitivity of the corrosion sensor may be adjusted by changing the thickness (h), chemical composition (affecting $D_0$), and/or processing conditions (affecting $D_0$) of the gas-permeable coating without changes in the dimensions of the corroding metal. This may lead to sensor elements with different measurable time lags, similarly, as for a corrosion sensor with steel resistor encapsulated under epoxy of different thickness.

As an additional feature, the gas permeable coating may exhibit gas selectivity in terms of ideal separation factor, see formula 1 below,

$$\alpha * (A/B) = \frac{\overline{D}(A)}{\overline{D}(B)} \cdot \frac{\overline{S}(A)}{\overline{S}(B)},$$

(1)

where $\overline{D}(A)/\overline{D}(B)$ and $\overline{S}(A)/\overline{S}(B)$ are the diffusivity selectivity and solubility selectivity, respectively, and where A is gas A, B is gas B, $\alpha^*$ (A/B) is ideal separation factor (between gases A and B), D(A) is mean diffusion coefficient of gas A, D(B) is mean diffusion coefficient of gas B, S(A) is solubility coefficient of gas A, and S(B) is solubility coefficient of gas B. In such a case, the corrosion sensor may provide additional information on the presence, absence, or relative concentration of certain corrosive gases. By selecting an array of similar readily corroding thin film metal patterns that are encapsulated under different gas-selective coatings, the presence of certain corrosive gases may be detected. This additional information may be used to identify the source(s) of the corroding gas(es).

An aspect of the invention is a corrosion sensor comprising a corroding metal thin film encapsulated with a polymer layer, wherein the polymer layer is permeable to a corrosive gas by diffusion, wherein an increase in the electrical resistance of the corrosion sensor, under exposure to corrosive gas that corrodes the corroding metal thin film when the corrosive gas permeates the polymer by diffusion, provides an indication of corrosion. The polymer may be an electrically insulating polymer, or at least the electrical conductivity of the polymer layer is significantly lower than that of the metal thin film.

In an embodiment, the corrosion sensor may be in the form of metal strip coated with polymer.

In an embodiment, the corroding metal may be Ag, Cu, Zn and/or Fe and/or any other metal that corrodes. The corroding metal is electrically conductive.

In an embodiment, the polymer may be poly-(ethylmethacrylate). In an embodiment, the polymer may be polydimethyl siloxane (PDMS), poly(1-trimethylsilyl-1-propyne) (PTMPS), or polycarbonate (PC), which are permeable to $H_2S$ and that are $CO_2/H_2S$ selective. In an embodiment, the polymer may be cellulose acetate, ethyl cellulose, poly(vinyl butyral), or polyethylene, which have high permeability to $H_2S$. In an embodiment, the polymer may be polyethylene terephthalate (PET, Mylar A), regenerated cellulose, poly(vinyl alcohol), poly(vinyl butyral/vinyl alcohol) copolymer, polyamide (Nylon-6), poly(vinylidene chloride), polypropylene, or poly(vinyl chloride), which are permeable to $H_2S$. In an embodiment, the polymer may be any combination of said polymers. The polymer may be different for adjacent sensors if selectivity is desired.

In an embodiment, the sensitivity of the corrosion sensor to the corrosive gas may be dependent on the layer thickness of the polymer layer in the corrosion sensor. The sensitivity of the corrosion sensor to the corrosive gas may increase with a decreased layer thickness of the polymer layer in the corrosion sensor. The sensitivity of the corrosion sensor to the corrosive gas may decrease with an increased layer thickness of the polymer layer in the corrosion sensor.

In an embodiment, the sensitivity of the corrosion sensor to the corrosive gas may be dependent on the gas permeability of the polymer layer in the corrosion sensor. The sensitivity of the corrosion sensor to the corrosive gas may increase with an increased gas permeability of the polymer layer in the corrosion sensor. The sensitivity of the corrosion sensor to the corrosive gas may decrease with a decreased gas permeability of the polymer layer in the corrosion sensor.

In an embodiment, the corrosion sensor may be in the form of a vacuum-deposited, patterned metal thin film encapsulated with spin-coated poly-(ethylmethacrylate) polymer.

In an embodiment, the corrosion sensor may be in the form of a patterned metal thin film obtained by slot-die coating, bar coating, blade coating, and/or vacuum deposition (evaporation, sublimation, atomic layer deposition (ALD), molecular layer deposition (MLD), chemical vapour deposition (CVD)), wherein the patterning may be formed by etching, lift-off, laser ablation, or using a physical mask layer during deposition.

In an embodiment, the metal thin film may be patterned using inkjet printing, flexography printing, gravure printing, and/or screen printing.

In an embodiment, the polymer coating may be deposited on the metal thin film by slot-die coating, bar coating, blade coating, printing (inkjet printing, flexography printing, gravure printing, and/or screen printing), and/or vacuum deposition (evaporation, sublimation, and/or ALD, MLD, CVD).

In an embodiment, the corrosion sensor may be fabricated directly onto a discrete electronic component (electronic component as the substrate) or integrated to an electronic circuit during the fabrication of the corrosion sensor (by deposition of metal and polymer onto prearranged contact pads on the electronic circuit).

In an embodiment, the corrosive gas may be at least one of $SO_z$ and $H_2S$.

In an embodiment, the layer thickness of the encapsulating polymer layer in the corrosion sensor may be from 10 nm to 10 μm. The time lag may be dependent on the square of the thickness, and if the coating is pinhole/defect-free, it may be possible to achieve different time lags (i.e. sensitivities) by scaling the thickness of the sensor.

In an embodiment, an initial thickness of the corroding metal thin film in the corrosion sensor may be in a range from 5 nm to 500 nm, or in a range from 50 nm to 500 nm. When during use the corrosion sensor/corroding metal thin film becomes partly or fully corroded, said thickness may decrease from that.

In an embodiment, the corrosion sensor may be used for monitoring the presence of a corrosive gas, wherein the corrosion sensor in the form of corrosive metal thin film encapsulated with an electrically insulating polymer layer. However, in addition/alternatively, conductive or semiconductive polymer layer may be used, if the electrical resistance of the polymer encapsulation is significantly higher than that of the corroding metal, i.e. the electrical conductivity of the polymer layer is significantly lower than that of the metal thin film.

In an embodiment, a method for monitoring presence of a corrosive gas is provided, the method comprising depositing or attaching the corrosion sensor on a substrate, subjecting the corrosion sensor to an atmosphere to be monitored, and measuring an increase of electrical resistance in the corrosion sensor. The substrate may be formed of a prefabricated pad and electronic circuitry for the integration of the sensor element. The substrate may be electrically connected to the external electric circuit through a connector, via wire-bonding, and/or with conductive glue. The prefabricated pads and electronic circuitry may be encapsulated with materials that are tolerant to the corrosive gas. Examples of materials that may be used to prevent effects of corrosive gases, include pinhole-free layers of metal oxide material(s), such as $Al_2O_3$, $TiO_2$, $SiO_2$, $HfO_2$ and/or nanolaminates thereof, and/or metal nitride material(s), such as TiN, TaN and/or WN, deposited using ALD.

In an embodiment, the sensitivity of the corrosion sensor may be tuned by using a polymer layer on one, two, three or four sides of the thin metal. Thus the sensitivity of the corrosion sensor may be adjusted by e.g. leaving one side of the sensor open for corrosion, i.e. the side of the metal conductor is not totally covered with polymer. For example, laser may be used to cut a slice of the polymer layer, leaving the side of the metal open.

In an embodiment, the corrosion sensor may be cut into a desired shape using laser technique.

An embodiment provides an electronic, low-cost corrosive gas sensor element that is able to provide in-situ information of corrosion phenomenon. The corrosion phenomenon is detectable as increasing electrical resistance after exposure of the corrosive gas sensor element to corrosive gases. Readily corrosive metal thin film, such as Ag or Cu, is encapsulated under a gas permeable coating, such as polymer, that allows permeation of certain corrosive gases. The resistance of the corrosion sensor element starts to increase when the corrosive gas has diffused through the gas permeable (polymer) coating.

An embodiment provides a tuneable corrosion sensor where a readily corroding metal as a thin film is encapsulated under a gas permeable coating, such as a polymer film. The electrical resistance of the corrosion sensor increases with exposure to corrosive gases that permeate the encapsulation. The corrosion sensor may be used in-situ to monitor corrosion behaviour. By changing the thickness, chemical composition, and processing conditions of the polymer encapsulation, the corrosion of the thin film metal may be restrained, under exposure to certain corrosive gases.

An embodiment enables providing low-cost, continuous monitoring of corrosive gases. An embodiment enables providing a replaceable corrosion sensor that may be replaced when the sensor becomes fully corroded or changes are made in the gas atmosphere. Additionally, an embodiment enables to selectively detect the presence of different corrosive gases by using polymer coatings with different gas permeability (ideal separation factor) or different polymer layer thicknesses, or by printing a polymer mask in optimal design on top of corroding metal layer.

An embodiment enables in-situ corrosion measurements that provide real time information of corrosion behavior at operation conditions.

In an embodiment, the increase of the electrical resistance in the corrosion sensor may be measured using an ER (electrical resistance) probe. The ER probe may be used to measure the change in Ohmic resistance of the corrosion sensor exposed to the corrosive gas(es). The action of corrosion on the corrosion sensor produces a decrease in its cross-sectional area with a corresponding increase in its electrical resistance. The increase in electrical resistance may be related directly to metal loss, and the metal loss as a function of time is the corrosion rate. By ER probes, direct corrosion rates may be obtained. They respond quickly to corrosion upsets and may be used to trigger an alarm. The (increase of) electrical resistance may be measured/detected using Ohm's law and supplying constant current (voltage) and measuring voltage drop over (current through) the corrosion sensor.

Example 1

A vacuum-deposited, patterned Ag thin film (Ag layer thickness of 30 nm) on a PET substrate was encapsulated with a spin-coated poly-(ethylmethacrylate) (PEMA) polymer that is permeable at least to $H_2S$. The onset of the corrosion in an atmosphere containing 1005 ppb $H_2S$ and 1005 ppb SOz was delayed about 40 days when compared to a reference Ag thin film without encapsulation.

Example 2

A 30 nm thick Ag layer was thermally evaporated in vacuum through a shadow mask onto a PET substrate to form 1 mm wide and 38 mm long Ag stripes the electrical resistance of which were monitored after exposure to corrosive gases. The initial sheet resistance of the Ag strip was 1.1 Ohm/sq.

Poly-(ethylmethacrylate) (PEMA) polymer is permeable to $H_2S$. PEMA with average molecular weight of about 515 kg/mol was dissolved in toluene in 3 wt% concentration to form an ink for spin-coating. By spin-coating PEMA ink at 2000 rpm onto the Ag sample, and drying the film on a hot plate at 110 °C for 1 min, a corrosion inhibiting layer was formed on top of the Ag strip. The initial sheet resistance of the PEMA-coated Ag strip was 1.3 Ohm/sq.

Ag thin films with and without PEMA coating (3 wt%) were exposed to corrosive gases $H_2S$ (at 1005 ppb) and SOz (at 1005 ppb) at 80% relative humidity. The electrical resistance of the bare Ag strip showed immediate increase with linearly increasing electrical resistance, whereas the onset of linear increase in the electrical resistance was delayed approximately 40 days for the sample with the PEMA coating. This demonstrates that an observable time lag was obtained using the PEMA coating. The test result are shown Figure 1. The results suggest that the corrosion of a readily corroding thin film metal under exposure to certain corrosive gases, can be restrained using the PEMA coating.

It will be obvious to a person skilled in the art that, as the technology advances, the inventive concept can be implemented in various ways. The invention and its embodiments are not limited to the examples described above but may vary within the scope of the claims.

**Claims**

1. A corrosion sensor comprising

   corroding metal thin film encapsulated or at least partly coated with a polymer layer, wherein the polymer layer is permeable to a corrosive gas by diffusion, wherein the electrical conductivity of the polymer layer is significantly lower than that of the metal thin film,
   wherein an increase in the electrical resistance of the corrosion sensor, under exposure to corrosive gas that corrodes the corroding metal thin film when the corrosive gas permeates the polymer layer by diffusion, provides an indication of corrosion.

2. The corrosion sensor according to claim 1, wherein it is in the form of metal strip encapsulated or at least partly coated with polymer.

3. The corrosion sensor according to claim 1 or 2, wherein the corroding metal is Ag, Cu, Zn and/or Fe and/or any other electrically conductive metal.

4. The corrosion sensor according to claim 1, 2 or 3, wherein the polymer is electrically insulating polymer.

5. The corrosion sensor according to any of the preceding claims, wherein the polymer is poly-(ethylmethacrylate), polydimethylsiloxane, poly(1-trimethylsilyl-1-propyne), polycarbonate, cellulose acetate, ethyl cellulose, poly(vinyl butyral), polyethylene, polyethylene terephthalate, regenerated cellulose, poly(vinyl alcohol), poly(vinyl butyral/vinyl alcohol) copolymer, polyamide, poly(vinylidene chloride), polypropylene, poly(vinyl chloride), or any combination thereof, preferably poly-(ethylmethacrylate).

6. The corrosion sensor according to any of the preceding claims, wherein the sensitivity of the corrosion sensor to the corrosive gas is dependent on the layer thickness of the polymer layer in the corrosion sensor,

   wherein the sensitivity of the corrosion sensor to the corrosive gas is increased with a decreased layer thickness of the polymer layer in the corrosion sensor,
   wherein the sensitivity of the corrosion sensor to the corrosive gas is decreased with an increased layer thickness of the polymer layer in the corrosion sensor.

7. The corrosion sensor according to any of the preceding claims, wherein the sensitivity of the corrosion sensor to the corrosive gas is dependent on the gas permeability of the polymer layer in the corrosion sensor,

   wherein the sensitivity of the corrosion sensor to the corrosive gas is increased with an increased gas permeability of the polymer layer in the corrosion sensor,
   wherein the sensitivity of the corrosion sensor to the corrosive gas is decreased with a decreased gas permeability of the polymer layer in the corrosion sensor.

8. The corrosion sensor according to any of the preceding claims, wherein it is in the form of vacuum-deposited, patterned metal thin film, encapsulated or at least partly coated with a spin-coated poly-(ethylmethacrylate) polymer.

9. The corrosion sensor according to any of the preceding claims, wherein the corrosive gas is at least one of $SO_z$ and $H_2S$.

10. The corrosion sensor according to any of the preceding claims, wherein a layer thickness of the polymer layer in the corrosion sensor is from 10 nm to 10 $\mu$m.

11. The corrosion sensor according to any of the preceding claims, wherein a thickness of the corroding metal thin film in the corrosion sensor is from 5 nm to 500 nm.

12. A use of the corrosion sensor according to any of claims 1 to 11 for monitoring presence of a corrosive gas, wherein the corrosion sensor is in the form of corroding metal thin film encapsulated or at least partly coated with a polymer layer.

13. A method for monitoring presence of a corrosive gas, the method comprising depositing a corrosion sensor according to any of claims 1 to 11 on a substrate, subjecting the corrosion sensor to an atmosphere to be monitored, and measuring an increase of electrical resistance in the corrosion sensor.

Figure 1

Europäisches
Patentamt

European
Patent Office

Office européen
des brevets

# EUROPEAN SEARCH REPORT

Application Number

EP 23 20 5809

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2009 250611 A (TAIYOSHA ELECTRIC CO LTD) 29 October 2009 (2009-10-29) <br> * figure 1 * <br> * paragraphs [0001], [0003], [0007], [0048] - [0049], [0058] * | 1-13 | INV. <br> G01N17/00 <br> G01N17/04 <br> G01N33/00 |
| A | US 11 262 289 B1 (OHODNICKI JR PAUL R [US] ET AL) 1 March 2022 (2022-03-01) <br> * figure 1C * <br> * column 6, line 9 - line 41 * | 1-13 | |
| A | US 2022/187231 A1 (SHELTON KATHERINE [US] ET AL) 16 June 2022 (2022-06-16) <br> * figures 1A-1C * <br> * paragraphs [0012] - [0016] * | 1-13 | |

TECHNICAL FIELDS
SEARCHED       (IPC)

G01N

The present search report has been drawn up for all claims

| Place of search | Date of completion of the search | Examiner |
|---|---|---|
| The Hague | 8 April 2024 | Bockstahl, Frédéric |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 20 5809

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2024

| Patent document cited in search report | | Publication date | Patent family member(s) | Publication date |
|---|---|---|---|---|
| JP 2009250611 | A | 29-10-2009 | NONE | |
| US 11262289 | B1 | 01-03-2022 | NONE | |
| US 2022187231 | A1 | 16-06-2022 | NONE | |

For more details about this annex : see Official Journal of the European Patent Office, No. 12/82